(19) Europäisches Patentamt
European Patent Office
Office européen des brevets

(11) **EP 3 788 957 B1**

(12) **EUROPEAN PATENT SPECIFICATION**

(45) Date of publication and mention of the grant of the patent:
**17.06.2026 Bulletin 2026/25**

(21) Application number: **19306071.2**

(22) Date of filing: **06.09.2019**

(51) International Patent Classification (IPC):
**A61B 5/0536** *(2021.01)*    **A61B 5/0537** *(2021.01)*
**A61B 5/107** *(2006.01)*    **A61B 5/00** *(2006.01)*

(52) Cooperative Patent Classification (CPC):
**A61B 5/1073; A61B 5/0536; A61B 5/0537; A61B 5/1075; A61B 5/4519**

(54) **METHOD, DEVICE AND APPARATUS FOR MEASURING SEGMENTAL MUSCLE VOLUME**

VERFAHREN, VORRICHTUNG UND EINRICHTUNG ZUR MESSUNG EINES SEGMENTALEN MUSKELVOLUMENS

PROCÉDÉ, DISPOSITIF ET APPAREIL DE MESURE DE VOLUME MUSCULAIRE SEGMENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.03.2021 Bulletin 2021/10**

(73) Proprietor: **Association Institut de Myologie 75013 Paris (FR)**

(72) Inventors:
- **HOGREL, Jean-Yves 92120 MONTROUGE (FR)**
- **BACHASSON, Damien 75012 PARIS (FR)**

(74) Representative: **IPAZ Parc Les Algorithmes Bâtiment Platon 91190 Saint Aubin (FR)**

(56) References cited:
**EP-A2- 1 712 179**

- **S. SALINARI ET AL: "New bioimpedance model accurately predicts lower limb muscle volume: validation by magnetic resonance imaging", AMERICAN JOURNAL OF PHYSIOLOGY: ENDOCRINOLOGY AND METABOLISM., vol. 282, no. 4, 1 April 2002 (2002-04-01), US, pages E960 - E966, XP055649521, ISSN: 0193-1849, DOI: 10.1152/ajpendo.00109.2001**
- **"Wiley Encyclopedia of Biomedical Engineering", 14 April 2006, JOHN WILEY & SONS, INC., Hoboken, NJ, USA, ISBN: 978-0-471-74036-0, article DAMIJAN MIKLAVCIC ET AL: "Electric Properties of Tissues", XP055649544, DOI: 10.1002/9780471740360.ebs0403**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Field of the invention**

The present invention relates to a method, a device and an apparatus for measuring segmental muscle volume of a human or an animal. The present invention falls under the field of medicine, biology and physiology. In particular, the invention is related to medical diagnostic.

**Background to the invention**

Skeletal muscle wasting is associated with negative outcomes across the healthcare continuum, e.g. lower physical function, higher rates of complications or comorbidities, poorer quality of life, and shorter survival.

Therefore, there is an important need for noninvasive and highly accurate methods for assessing skeletal muscle mass/volume. The assessment of the skeletal muscle mass/volume is essential in general population and conditions involving primary and/or secondary muscle wasting and degeneration. The skeletal muscle mass/volume may be an indicator of primary and/or secondary muscle wasting and degeneration.

One knows in the state of the art, assessment of skeletal muscle mass through technics such as whole body counting with neutron activation, dual-energy X-ray absorptiometry, computerized tomography, and nuclear magnetic resonance imaging. These methods are currently considered as standard methods for skeletal muscle mass/volume measurement.

These methods are expensive, time consuming, require high level of expertise and require specific and cumbersome facilities in which the patient has to be brought.

One knows bioelectrical impedance analysis (BIA) as detailed in the documents Salinari et al., Journal of Applied Physiology, vol.94: 1552-1556, 2003 and Salinari et al., American Journal of Physiology Endocrinology and Metabolism, 282: E960-6, 2002. However, current BIA methods exhibit critical limitations.

The assessment of the skeletal muscle mass through conventional BIA methods is not accurate. Indeed, the models used for the assessment are oversimplified to embrace the physiological between individual differences.

Moreover, the models are highly population specific, making them poorly generalizable.

**Summary of the invention**

An object of the invention is:

- to provide a method that overcomes at least one of the drawbacks of the methods of the state of the art, and/or
- to provide a non-invasive method that may be applied to all type of populations, and/or
- to provide a method that can be carried out at patient's bedside, and/or
- to provide a method that is accurate and non-population specific.

To this end, according to the invention, there is provided a method for determining a muscle section of a body part in which the muscle section (S) is determined as a function of:

- at least two electrical resistance values measured through different electrodes located on different locations of the body, at least two electrodes being located along said body part, and
- a muscle conductivity constant ($\sigma$), and
- a permittivity constant of the muscle ($\varepsilon$).

The at least two electrodes located along the body part are preferably comprised among the different electrodes located on the different locations of the body.

The method may comprise a step of measuring and/or determining the at least two electrical resistance values from the different electrodes located on different locations of the body. The step of determining the at least two electrical resistance values may be carried out by a processing unit.

The at least two electrodes located along the body part are preferably located at different locations of the body part separated from each other's from known distances.

According to the invention, at least two electrical reactance values may be measured in combination with or as a substitute to the at least two electrical resistance values. The at least two electrical resistance values according to the invention may be substituted by or combined with the at least two electrical reactance values.

The at least two electrical resistance values may be measured by bioelectrical impedance analysis.

In the present document, when the term "electrode" is used alone, it designates a voltage electrode.

The different electrodes located on different locations of the body may comprise at least three electrodes; at least one electrode of the at least three electrodes being located on a part of the body that is different from the body part of which the muscle section (S) is determined and two of the at least three electrodes being located along the body part.

The method may comprise a measurement of an electrical potential at each of the at least two electrodes being located along said body part. The at least two electrodes being located along said body part may be part of an electrodes array adapted for measuring electrical potentials. Preferably, the electrodes array for measuring electrical potentials is positioned along the body part. The electrodes of the electrodes array for measuring electrical potentials may be adapted to measure a variation of electrical potential along the body part; the variation of electrical potential along the body part being induced by a current that flows through the body part.

The method may comprise injecting a current that flows through the body part from a current injection electrode towards another current injection electrode among two current injection electrodes forming a pair of current injection electrodes. The current that is injected may be an alternative current. The alternative current may be injected at a single pulsation or successively at several different pulsations. Preferably, one among the two current electrodes may be located onto the body part and one among the two current electrodes may be located on a part of the body that is different from the body part of which the muscle section (S) is determined.

The method may comprise a step of determining a variation of the electrical resistance between two electrodes among the at least two electrodes located along the body part, and consequently along the body part.

The muscle section may be determined as a function of a term of conductivity comprising, for each considered electrical resistance value, a product of the considered electrical resistance value and the muscle conductivity constant.

The muscle section may be determined as a function of a corrective term.

The corrective terms may allow correcting a standard term currently used for the determination of the muscle section.

The standard term currently used for the determination of the muscle section may be the product of the electrical resistance value and the muscle conductivity constant.

The corrective term may comprise a product of the square of the measurement frequency and the square permittivity constant of the muscle. The measurement frequency may be directly related and/or proportional to and/or equal to the pulsation of the alternative current injected between the two current injection electrodes.

The corrective term may comprise, for each considered electrical resistance value, a product of the considered electrical resistance value and the square permittivity constant of the muscle.

The corrective term may comprise, for each considered electrical resistance value, a ratio of the considered electrical resistance value and the muscle conductivity constant.

The corrective term may comprise, for each considered electrical resistance value, a product of the considered electrical resistance value and the square of the measurement frequency.

The muscle section may be determined as a function of:

- a sum of the term of conductivity and the corrective term, and/or
- the inverse of the sum of the term of conductivity and of the corrective term.

The muscle section may be determined from:

- at least a pair of electrical resistance values being measured at a single measurement frequency or at different measurement frequencies, and/or
- several pair of electrical resistance values; at least one pair of electrical resistance values is measured at a measurement frequency that is different from a measurement frequency at which another pair of electrical resistance values is measured, and/or
- several pair of electrical resistance values; at least one pair of electrical resistance values is measured at a measurement frequency that is different from each measurement frequency at which each other respective pair of electrical resistance values is measured;

each pair of electrical resistance values preferably comprising a first electrical resistance value measured at a first electrode located at a first location of the body part and a second electrical resistance value measured at a second electrode located at a second location of the body part.

A pair of electrical resistance values is preferably comprised among the at least two electrical resistance values measured through different electrodes located on different locations of the body.

Each electrical resistance value of a pair of electrical resistance values is preferably comprised among the at least two electrical resistance values measured.

The first and the second electrodes are preferably comprised among the at least two electrodes being located along the body part.

A relative electrical resistance value may be determined from the at least two electrical resistance values measured.

The relative electrical resistance value may be determined from one pair of electrical resistance values. The relative electrical resistance value may be the resistance of the body part between the first electrode and the second electrode.

The first or the second locations of the body part at which respectively the first and the second electrical resistance values of a pair of electrical resistance values are measured may be identical to a first or a second locations of the body part at which respectively a first and a second electrical resistance values of another pair of electrical resistance values are measured.

According to the invention, the muscle section (S) is determined as a function of formula 1,

$$S = \frac{1}{\frac{\delta R}{\delta z}.(\sigma + \frac{\varepsilon^2 w^2}{\sigma})}, \text{ formula 1,}$$

in which $\frac{\delta R}{\delta z}$ is an electrical resistance gradient between two locations of the body part separated from a distance z and $\omega$ is the pulsation at which the at least two electrical resistance values are measured, the electrical resistance gradient $\frac{\delta R}{\delta z}$ being determined from said at least two electrical resistance values.

The two locations of the body part, separated from a distance z, between which the electrical resistance gradient is determined, may be the first and the second locations at which respectively the first and the second electrodes are located.

The electrical resistance gradient $\frac{\delta R}{\delta z}$ may be determined from a pair of electrical resistance values.

The muscle conductivity constant and/or the permittivity constant of the muscle may be a function of the measurement frequency at which the at least two electrical resistance values are measured; said muscle conductivity constant being comprise between 0.1 and 2 Siemens/meter (S/m), preferably between 0.4 and 1.5 S/m, more preferably between 0.6 and 1.2 S/m; and said permittivity constant of the muscle being comprise between $1.10^{-8}$ and $1.10^{-6}$ Farad/meter (F/m), more preferably between $5.10^{-8}$ and $7.10^{-7}$ F/m.

More preferably:

- the muscle conductivity constant is comprised between 0.6 and 1.1 S/m in the range of measurement frequencies comprise between 40 and 60 kHz, and/or
- the muscle conductivity constant increases to be comprised between 0.7 and 1.2 S/m in the range of measurement frequencies comprise between 220 and 260 kHz, and/or
- the muscle conductivity constant decreases to be comprised between 0.65 and 1.15 S/m in the range of measurement frequencies comprise between 340 and 360 kHz.

More preferably:

- the permittivity constant of the muscle is comprised between $7.10^{-7}$ and $3.7.10^{-7}$ F/m in the range of measurement frequencies comprise between 40 and 60 kHz, and/or
- the permittivity constant of the muscle decreases to be comprised between $3.5.10^{-7}$ and $1.10^{-7}$ F/m in the range of measurement frequencies comprise between 180 and 220 kHz, and/or
- the permittivity constant of the muscle decreases to be comprised between $3.10^{-7}$ and $5.10^{-8}$ F/m in the range of measurement frequencies comprise between 340 and 360 kHz.

The muscle conductivity constant $\sigma$ may be defined as a function of the measurement frequency f according to formula 2:

$$\sigma = af^2 + bf + c, \text{ formula 2,}$$

and/or the permittivity constant $\varepsilon$ may be defined as a function of the measurement frequency f according to formula 3:

$$\varepsilon = d.e^{-gf} + h.e^{-kf}, \text{ formula 3,}$$

where the terms a, b, c, d, g, h and k of the equations may be function of the frequency f or may be constants.

The measurement frequency may be comprised between 40 and 1000 kHz.

Preferably, the measurement frequency may be comprised between 50 and 500 kHz.

More preferably, the measurement frequency may be comprised between 100 and 300 kHz.

According to the invention, there is also provided a method for determining a muscle volume of a body part, the muscle volume of the body part may be determined from one or more muscle sections of the body part, each muscle section of the body part being determined according to invention.

The muscle volume of the body part may be calculated by integrating the muscle sections over a distance z separating two electrodes farther apart among the at least two electrodes being located along said body part.

One may also determine the volume of a portion of a body part. The volume of a portion of a body part may be calculated by integrating one or more muscle sections over a distance z separating the at least two electrodes being located along said body part between which the muscle section is determined.

The muscle volume (V) of a body part or of a portion of a body part may be determined according to formula 4,

$$v = \int S_n(z)dz, \text{ formula 4,}$$

in which n is the number of sections $S_n$ extending along a distance z between the at least two electrodes being located along said body part and between which the muscle section S is determined. The number of sections n is an integer greater than or equal to 1.

According to the invention, there is also provided an apparatus comprising technical means arranged for and/or configured to and/or programmed to carry out the method according to invention.

According to the invention, there is also provided a computer program product comprising instructions which, when the program is executed by a computer, cause the computer to carry out the method according to the invention.

## Brief description of the drawings

Further objects, features and advantages will appear from the following detailed description of several embodiments of the invention with references to the drawings, in which:

- FIGURE 1 is a schematic representation of the experimental setup used to measure electrical resistance values on human participants,
- FIGURE 2 is a graph illustrating the electrical resistance values measured at each voltage electrodes of the electrodes array located along the thigh of a human subject as a function of the measurement frequency,
- FIGURE 3 is a graph illustrating the electrical resistance gradient as a function of the distance separating the voltage electrodes of the electrodes array arranged along the thigh muscle of a human subject,
- FIGURE 4 is a graph illustrating the muscle conductivity determined according to the invention as a function of the measurement frequency,
- FIGURE 5 is a graph illustrating the permittivity constant of the muscle determined according to the invention as a function of the measurement frequency,
- FIGURE 6 is a graph illustrating the muscle section determined according to the invention and determined through nuclear magnetic resonance imaging as a function of the distance along the muscle,
- FIGURE 7 is a graph illustrating the muscle section determined according to the invention at the middle of the muscle of each person among a group of human subjects versus the muscle section determined through nuclear magnetic resonance imaging at the middle of the muscle of each person among the group of human subjects,
- FIGURE 8 is a graph illustrating the muscle volume determined according to the invention on the group of human subjects versus the muscle volume determined through nuclear magnetic resonance imaging on said group of human subjects.

## Detail description of embodiments of the invention

The embodiments hereinafter described are not restrictive, other embodiments comprising a selection of features described hereinafter may be considered. A selection may comprise features isolated from a set of features (even if this selection is isolated among a sentence comprising other features thereof), if the selection is sufficient to confer a technical advantage or to distinguish the invention form the state of the art. This selection comprises at least a feature, preferably described by its technical function without structural features, or with a part of structural details if this part is sufficient to confer a technical advantage or to distinguish the invention form the state of the art on its own.

Furthermore, the embodiments hereinafter are non-limitative embodiments that are within the scope of the above summary of the invention. Thus, any isolated feature of the below embodiments is considered in combination with the more general or functional steps or features of the above summary of the invention. The invention is defined by the appended claims.

Participants

A group of 20 human subjects comprising 8 healthy participants and 11 patients. The 11 patients exhibited neuromuscular disorders associated with muscle wasting. All participants gave written informed consent.

According to the invention, a muscle section of a body part is determined as a function of:

- at least two electrical resistance values measured through different electrodes located on different locations of the body, at least two electrodes being located along said body part, and
- a muscle conductivity constant $\sigma$, and
- a permittivity constant of the muscle $\varepsilon$.

The step of determining the muscle section is carried out by the processing unit of a computer. The muscle section is also know by a skilled person as contractile Cross Section Area (cCSA).

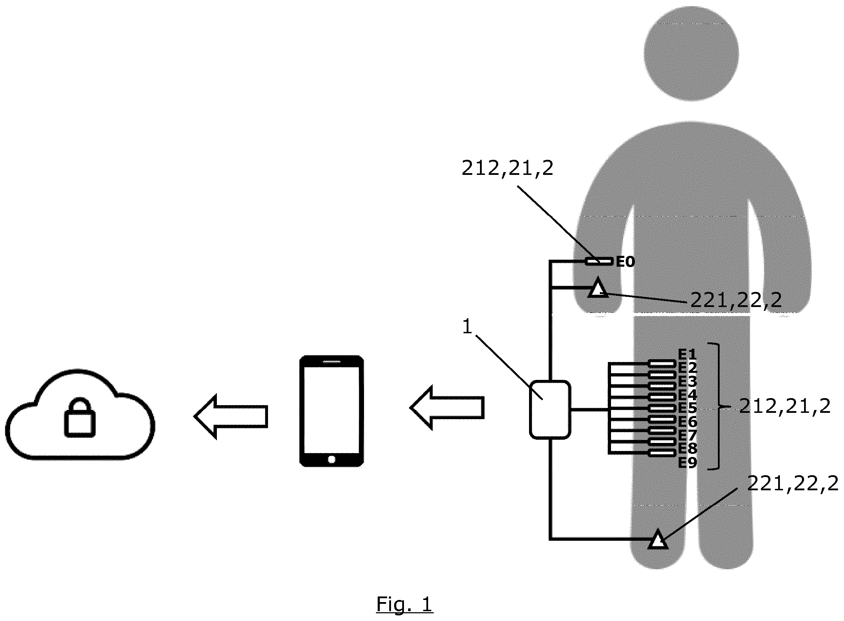

The Figure 1 shows a schematic representation of the experimental setup used on the human participants to perform the measurements. The setup is arranged to carry out bioelectrical impedance measurements in order to measure at least two electrical resistance values.

The setup comprises a multifrequency bioelectrical impedance analyzer 1 sold under the trade name Zmetrix by the manufacturer Bioparhom. The step of determining the at least two electrical resistance values is carried out by the processing unit of the analyzer. The setup comprises twelve different electrodes 2 located on different locations of the body of a subject. Among these twelve electrodes, ten electrodes are voltage electrodes 21 arranged to measure an electrical potential and two electrodes are current electrodes 22 arranged to inject an alternative current comprises between 50 and 100 $\mu$A that flows through the body of the subject. The electrodes are skin electrodes placed on the skin of the subject along the long axis of tested segment. The injected current is injected either at a single pulsation or at several different pulsations as appropriate. The measurement frequency is directly related to the pulsation of the alternative current injected between the two current injection electrodes.

Among the ten voltage electrodes 21, nine electrodes 21 form an electrodes array 211 arranged along the thigh of which the muscle section (S) is determined according to the invention describes herein under. The nine electrodes 21 of the electrodes array 211 are numbered from E1 to E9 and are noted Em, m being the number of the considered electrodes 21 of the electrodes array 211. The electrodes E1,21 of the electrodes array 211 is located on the side of the chest of the patient's thigh and the electrodes E9,21 of the electrodes array 211 is located on the side of the calf of the patient's thigh.

The voltage electrodes 21 of the electrodes array 211 are separated from each other's from known distances. The last voltage electrode E0,21 is arranged on a part of the body, on the hand according to the embodiment, which is different from the thigh. One of the current electrodes 222 is located on the ankle of the subject and the other 221 is located on the hand of the subject so that the current flows at least through the thigh of the subject.

Although, the electrodes array 211 according to the embodiment comprises nine electrodes, the electrodes array may comprise a different number of electrodes as appropriate. For instance, the electrodes array 211 may be substituted by only two voltage electrode 21 located at two different locations of the muscle of the subject.

In practice, the muscle section is determined from at least a pair of electrical resistance values. Each electrical resistance value of a pair of electrical resistance values is comprised among the at least two electrical resistance values measured. Each electrical resistance value is measured by a pair of voltage electrodes 21. A pair of voltage electrodes 21 measures the electrical resistance value between the two electrodes 21 of the pair of voltage electrodes 21. According to the embodiment, each pair of voltage electrodes 21 comprises the voltage electrode E0,21 located on the hand of the patient and one voltage electrode Em,21 of the electrodes array 211.

The voltage electrode E0,21 located on the hand of the patient is used as a reference electrode so that each electrical resistance value may be determined from a pair of voltage electrodes 21 comprising the voltage electrode E0,21 located on the hand and one voltage electrode Em,21 of the electrodes array 211. Thus, each electrical resistance value measured is considered as the electrical resistance value $R_{E_m\text{-}E_0}$ measured at the location of the thigh at which the voltage electrode Em,21 of the electrodes array 211 is placed.

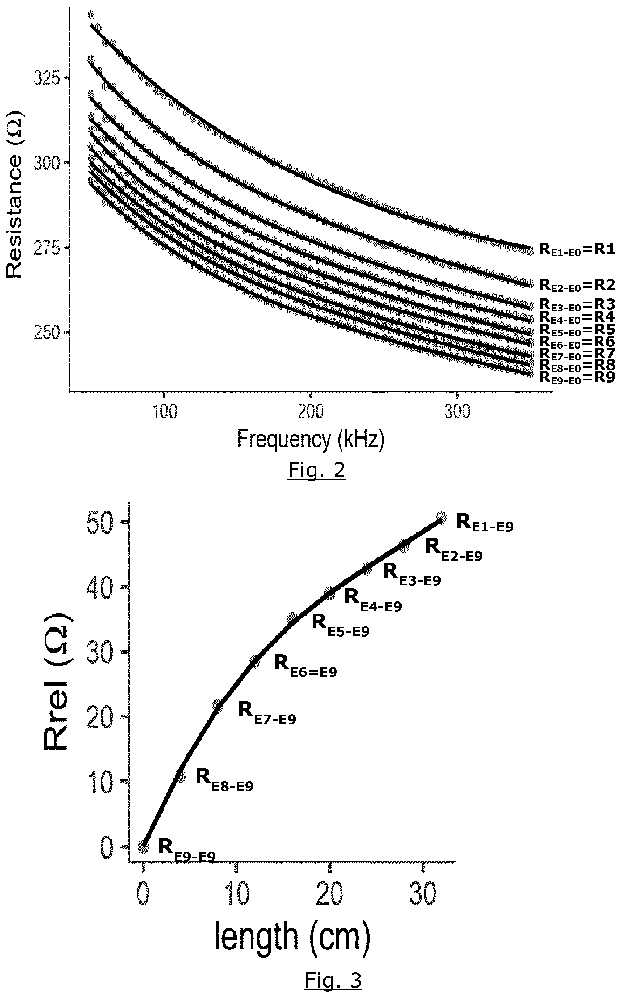

In reference to FIGURE 2, it is presented a graph illustrating the electrical resistance values $R_{E_m\text{-}E_0}$ measured at each voltage electrodes Em,21 of the electrodes array 211 located along the thigh of a human patient as a function of the measurement frequency f. The measurement frequency f is comprised between 50 and 350 kHz, with 10 kHz interval.

Using a Levenberg-Marquardt algorithm to perform nonlinear regression, the inventors notice that a double exponential function fit the measurement with a good agreement as supported by a coefficient of determination of 0.997 $\pm$ 0.018.

Each pair of electrical resistance values comprises a first electrical resistance value $R_{E_{m_1}-E_0}$ measured at a first voltage electrode $Em_1$,21 of the electrodes array 211 that is located at a first location of the thigh and a second electrical resistance value $R_{E_{m_2}-E_0}$ measured at a second voltage electrode $Em_2$,21 of the electrodes array 211 that is located at a second location of the thigh. The first and the second locations of the thigh are separated from a distance z between which an electrical resistance gradient $\frac{\delta R}{\delta z}$ may be determined. According to the embodiment, the second electrical resistance value $R_{E9-E_0}$ measured at the second location of the thigh is the voltage electrode E9,21 of the electrodes array 211.

A relative electrical resistance value Rm is defined as the resistance of the thigh between a pair of electrodes 21 comprising a first electrode Em,21 and the second electrode E9,21. Namely, the relative electrical resistance value Rm is the resistance of the thigh between the location of the first electrode Em,21 of the pair of electrodes 21 and the location of the second electrode E9,21 of the pair of electrodes 21. In other words, the relative electrical resistance value Rm is the resistance of a portion of the thigh according to the distance z separating the electrodes 21 of the pair of electrodes 21 of the electrodes array 211.

The relative electrical resistance value Rm is determined from the formula 5,

$$Rm = R_{E_m-E_0} - R_{E_9-E_0}, \text{ Formula 5.}$$

The relative electrical resistance values Rm are comprised between $R1 = R_{E1-E_0} - R_{E9-E_0}$ and $R8 = R_{E8-E_0} - R_{E9-E_0}$. Then, an electrical resistance gradient $\frac{\delta R}{\delta z}$ between the first and the second locations separated from a distance z may be determined from at least two of the relative electrical resistance values Rm. The electrical resistance gradient $\frac{\delta R}{\delta z}$ shows the variation of the electrical resistance, and of the electrical potential, along the thigh.

The FIGURE 3 shows the relative electrical resistance gradient $\frac{\delta R}{\delta z}$ as a function of the distance z separating two voltage electrodes Em,21 of the electrodes array 211 arranged along the thigh muscle.

Using an adaptive nonlinear least-square algorithm to perform nonlinear regression, the inventors notice that a third degree polynomial function fit the measurement with a good agreement as supported by a coefficient of determination of 0.981 ± 0.082.

It worth to note that $Rm.\Delta I_{inj} = \Delta Vm$, where $\Delta I_{inj}$ is the injected current and $\Delta Vm$ is the difference of electrical potential measured between the first voltage electrode Em,21 located at a first location of the thigh and the second voltage electrode E9,21 positioned at a second location of the thigh. Considering that $Rm = R_{E_m-E_0} - R_{E9-E_0}$, then $\Delta Vm = V_{Em} - V_{E0} - V_{E9} + V_{E0}$. Thus, with an appropriate calibration, $V_{E0}$ may be a predefined constant which will allow to directly measuring the electrical resistance gradient $\frac{\delta R}{\delta z}$ solely from the measurements of two voltage electrodes 21 of the electrodes array 211 arranged along the thigh.

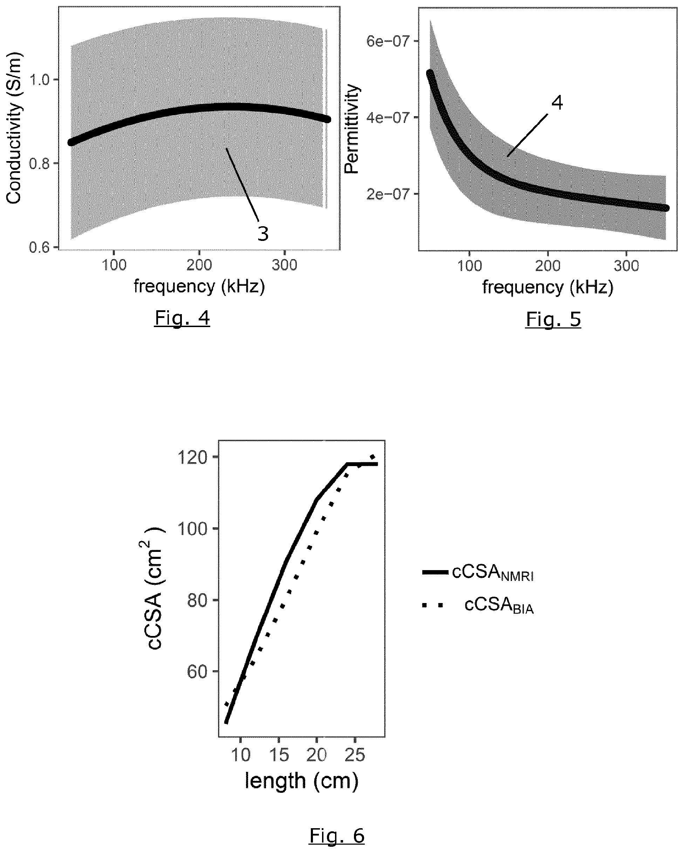

In reference to FIGURE 4, the muscle conductivity constant $\sigma$ of a muscle is determined according to the invention as a function of the measurement frequency. The inventors use formula 6 to determine the muscle conductivity constant $\sigma$ from muscle sections S of the thigh determined through nuclear magnetic resonance imaging in healthy participants (right side only) of the group, from reactance values X measured by the analyzer between pair of voltage electrodes Em,21 of the electrodes array 211 used for the measurement, from electrical resistance values $R_{E_m-E0}$ measured by the analyzer between pair of voltage electrodes Em,21 of the electrodes array 211 and from the distance z separating two voltage electrodes Em,21 of a pair of electrodes 21 of the electrodes array 211 arranged along the thigh muscle,

$$\sigma = \frac{z}{\left(R + \frac{X^2}{R}\right).S}, \text{ formula 6.}$$

The inventors notice that a second degree polynomial function according to formula 2 fits the muscle conductivity constant $\sigma$ with a good agreement as supported by a coefficient of determination of 0.995:

$$\sigma = af^2 + bf + c, \text{ formula 2,}$$

in which a, b and c are constants, a being equal to $-2.41.10^{-12}$, b is being equal to $1.15.10^{-6}$ and c being equal to 0.8.

The black line shows the mean value of the muscle conductivity constant $\sigma$ according to the measurement frequency and the area 3 shows the Confidence Interval (CI). The differences between the muscle conductivity constant $\sigma$ values are due to the variations of the muscle conductivity constant $\sigma$ resulting from the effect of the measurement frequency and the physiological between individual differences.

The conductivity constant $\sigma$ is comprised between 0.6 and 1.2 Siemens/meter (S/m) in the area 3. Given the fact that measurements has been carried out on a small sample of human subjects, the conductivity constant $\sigma$ may be considered to be comprised between 0.1 and 2 S/m in absolute terms. The muscle conductivity constant $\sigma$ is comprised between 0.6 and 1.1 S/m in the range of measurement frequency comprises between 40 and 60 kHz. The muscle conductivity constant $\sigma$ then increases to be comprised between 0.7 and 1.2 S/m in the range of measurement frequency comprises between 220 and 260 kHz. The muscle conductivity constant then decreases to be comprised between 0.65 and 1.15 S/m in the range of measurement frequency comprises between 340 and 360 kHz.

In reference to FIGURE 5, the inventors determined the permittivity constant of the muscle $\varepsilon$ according to the invention as a function of the measurement frequency. The inventors use formula 7 to determine the permittivity constant of the muscle $\varepsilon$ from the reactance gradient $\frac{\delta X}{\delta z}$ as a function of the distance z separating two voltage electrodes Em,21 of the electrodes array 211 arranged along the thigh muscle, from the muscle conductivity constant $\sigma$ determined as described above, from the pulsation $\omega$ of the measurement, and from the relative electrical resistance gradient $\frac{\delta R}{\delta z}$ as a function of the distance z separating two voltage electrodes Em,21 of the electrodes array 211 arranged along the thigh muscle,

$$\varepsilon = \frac{\sigma . \frac{\delta X}{\delta z}}{\omega . \frac{\delta R}{\delta z}}, \text{ formula 7,}$$

in which $\omega = 2\pi f$.

The black line shows the mean value of the permittivity constant of the muscle $\varepsilon$ according to the measurement frequency and the area 3 shows the Confidence Interval (CI). The differences between the permittivity constant of the muscle $\varepsilon$ values are due to the variations of the permittivity constant of the muscle $\varepsilon$ resulting from the effect of the measurement frequency and the physiological between individual differences.

The inventors notice that a double exponential function according to formula 3 fits the permittivity constant $\varepsilon$ with a good agreement as supported by a coefficient of determination of 0.999:

$$\varepsilon = d.e^{-gf} + h.e^{-kf}, \text{ formula 3,}$$

in which d, g, h and k are constants, d being equal to $1.012.10^{-6}$, g being equal to -10.54., h being equal to $2.641.10^{-7}$ and k being equal to -13.49.

The permittivity constant $\varepsilon$ is comprised between $5.10^{-8}$ and $7.10^{-7}$ Farad/meter (F/m) in the area 4. Given the fact that measurements has been carried out on a small sample of human subjects, the permittivity constant $\varepsilon$ may be considered to be comprised between $1.10^{-8}$ and $1.10^{-6}$ F/m in absolute terms. The permittivity constant $\varepsilon$ of the muscle is comprised between $7.10^{-7}$ and $3.7.10^{-7}$ F/m in the range of measurement frequency comprises between 40 and 60 kHz. The permittivity constant $\varepsilon$ of the muscle then decreases to be comprised between $3.5.10^{-7}$ and $1.10^{-7}$ F/m in the range of measurement frequency comprises between 180 and 220 kHz. The permittivity constant $\varepsilon$ of the muscle decreases again to be comprised between $3.10^{-7}$ and $5.10^{-8}$ F/m in the range of measurement frequency comprises between 340 and 360 kHz.

The measurements frequencies are comprised between 50 and 350 kHz. The inventors observe that measurements frequencies lesser than 100 kHz may lead to unreliable results because these frequencies are expected to be insufficient to penetrate the intracellular space and produce inconsistency related to high variation in skin-electrode impedance. On the opposite, measurements frequencies higher than 350 kHz, even 300 kHz, may lead to unreliable results because these frequencies highlights phenomenon at interfaces and phenomenon of diffusion which dominate from this frequencies.

The muscle section is determined as a function of the inverse of a sum of the term of conductivity and of a corrective term. The standard term is the term currently used for the determination of the muscle section. It is the product of the electrical resistance values and the muscle conductivity constant. The corrective terms aims to correct the standard term currently used for the determination of the muscle section. The standard term may be noted ( $\frac{\delta R}{\delta z}.\sigma$ ).

The purpose of the corrective term is to weight the effect of the measurement frequency on the muscle conductivity constant and on the permittivity constant of the muscle. The corrective term also aims to moderate the variance of the electrical resistance value induced by the physiological between individual differences. The corrective term also weights the effect of the measurement frequency on the variance of the electrical resistance value induced by the physiological between individual differences.

The muscle section (S) is determined as a function of formula 1,

$$S = \frac{1}{\frac{\delta R}{\delta z}.(\sigma + \frac{\varepsilon^2 w^2}{\sigma})}, \text{ formula 1,}$$

in which $\frac{\delta R}{\delta z}$ is an electrical resistance gradient between two locations of the body part separated from a distance z and $\omega$ is the pulsation at which the at least two electrical resistance values are measured, the electrical resistance gradient $\frac{\delta R}{\delta z}$ being determined from said at least two electrical resistance values.

In reference to FIGURE 6, it is shown a graph wherein the solid line illustrates the muscle section determined according formula 6 from bioimpedance measurements carried out along the thigh muscle of a healthy subject. It is also shown a dot line illustrating the muscle section determined through nuclear magnetic resonance imaging (NMRI) along the thigh muscle of the healthy subject. NMRI is considered to be a reference method for skeletal muscle mass measurement. The bioelectrical impedance measurement is carried out at 150 kHz and the values of the muscle conductivity constant $\sigma$, 0.916 S/m, and the permittivity constant $\varepsilon$, 2.338 e10$^{-7}$ F/m, used for the determination are respectively from FIGURES 4 and 5. As illustrated in FIGURE 6, the section determined by the invention is consistent with the section determined by NMRI, the standard error of measurement is about 5.94 %.

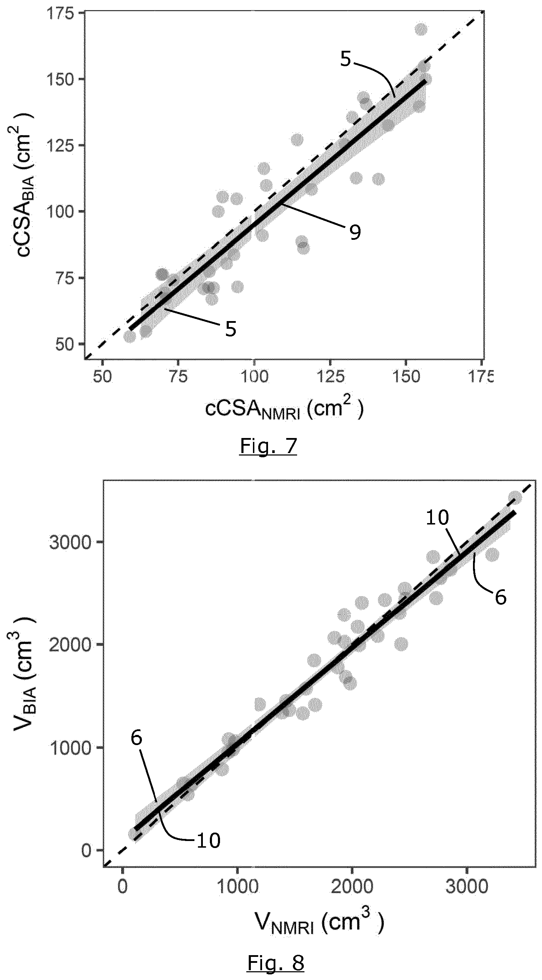

In reference to FIGURES 7 and 8, the dots illustrate the muscle section, or the muscle volume calculated from the muscle section, of each person among the group subjects (healthy participants and patients), the muscle section being determined through formula 1 from bioelectrical impedance measurements carried out at the middle of the thigh of each person of the group versus the muscle section determined at the middle of the thigh of each person of the group through nuclear magnetic resonance imaging. The bioimpedance measurement is carried out at 150 kHz and the values of the muscle conductivity constants, 0.916 S/m and the permittivity constant $\varepsilon$, 2.338 e10$^{-7}$ F/m, used for the determination are respectively from FIGURES 4 and 5. The ordinate axis represents the muscle section determined according to the invention and the abscissa axis represents the muscle section determined through nuclear magnetic resonance imaging. The straight lines 9 and 10 are obtained through the fit by linear regression of the muscle section values determined. The areas 5 and 6 show the Confidence Interval (CI) of the values of the muscle section.

TABLES 1 and 2 are related to the statistical parameters of the linear regression respectively of the muscle section values and of the muscle volume values as shown on FIGURES 7 and 8 respectively.

TABLES 1 and 2 show the muscle sections (S) at mid-thigh and the muscle volume (V) determined from the invention (cCSA$_{BIA}$ (cm$^2$) and V$_{BIA}$ (cm$^3$), respectively) and from NMRI (cCSA$_{NMRI}$ (cm$^2$) and V$_{NMRI}$ (cm$^3$), respectively), the p-value of the Student's t-test (a two-sample location test of the null hypothesis such that the means of two populations are equal), the Change In Mean with 95% confidence interval (CIM(cm$^2$) [95% CI]), the Standard Error of Measurement expressed as a coefficient of variation with 95% confidence interval (SEM(%) [95% CI]), and the Inter-Class Correlation coefficient with 95% confidence interval ([95% CI])) between cCSA$_{BIA}$ and cCSA$_{NMRI}$ (TABLE 1) and between V$_{BIA}$ and V$_{NMRI}$ (TABLE 2).

TABLE 1

| cCSA$_{NMRI}$ (cm$^2$) | cCSA$_{BIA}$ (cm$^2$) | P value | CIM(cm$^2$) [95% CI] | SEM(%) [95% CI] | ICC [95% CI] |
|---|---|---|---|---|---|
| 105.71 ±29.44 | 100.41 ±31.07 | <0.05 | -5.29 [-9.60, -0.98] | 8.59 [6.97, 11.21] | 0.9 [0.81, 0.95] |

TABLE 2

| V$_{NMRI}$ (cm$^3$) | V$_{BIA}$ (cm$^3$) | P value | CIM(cm$^3$) [95% CI] | SEM(%) [95% CI] | ICC [95% CI] |
|---|---|---|---|---|---|
| 1791.11 ±786.17 | 1773.69 ±755.88 | 0.0566 | -17.43 [-78.39, -43.54] | 9.03 [7.36, 11.68] | 0.97 [0.95, 0.99] |

In reference to FIGURE 7 and TABLE 1, one can notice the good agreement of the muscle section determined through the invention and the muscle section determined by NMRI. This result shows excellent agreement of the measurement of lean muscle section using the present invention with standard nuclear magnetic resonance imaging.

According to the invention, there is also provided a method for determining a muscle volume of a body part from one or more muscle sections of the body part, each muscle section of the body part being determined according to invention. According to the embodiment, the muscle volume (V) of a section of the thigh is determined according to formula 4,

$$v = \int S_n(z)dz, \text{ formula 4,}$$

in which n is the number of sections $S_n$ extending along a distance z between two voltage electrodes Em,21 of the electrodes array 211 along the thigh and between which the muscle section S is determined. The number of sections n is an integer greater than or equal to 1.

The muscle volume of the thigh as a whole is calculated by integrating the muscle section over the distance z separating the two electrodes farther apart among the voltage electrodes Em,21 of the electrodes array 211, namely E1 and E9.

In reference to FIGURE 8, it is illustrated the muscle volume of the thigh determined from muscle section determined according to the invention versus the muscle volume determined from muscle section determined through nuclear magnetic resonance imaging. The dots illustrate the muscle volume of the thigh determined in healthy participants and patients. The muscle volume is determined from the muscle section through formula 4. The straight line 10 is obtained through the fit by linear regression of the muscle section values of the thigh determined in the healthy subjects of the group and in the patients of the group. The area 6 shows the CI of the values of the muscle section values of the thigh determined in the healthy subjects in the patients of the group.

In reference to FIGURE 8 and TABLE 2, one can notice the good agreement of the muscle volume determined through the invention and the muscle section determined by NMRI. This result shows excellent agreement of the measurement of lean muscle section using the present invention with standard nuclear magnetic resonance imaging.

The invention is not restricted to embodiments described above and numerous adjustments may be achieved within the scope of the invention.

Moreover, features, alternatives and embodiments of the invention may be associated if they are not mutually exclusive of each other.

Thus, in combinable alternatives of previous embodiments:

- the body part of which the muscle section (S) is determined through the method according to the invention is the calf or the forearm or the upper arm, and/or
- at least two electrical reactance values are measured by the analyzer in combination with or as a substitute to the at least two electrical resistance values, and/or
- the at least two electrical resistance values according to the invention may be substituted by or combined with the at least two electrical reactance values using formula 8,

$$Z_{imp} = R + jX, \text{ formula 8,}$$

in which $Z_{imp}$ is the impedance, R is the resistance and X is the reactance, and/or
- the muscle section (S) is determined from pairs of electrical resistance values measured at several different measurement frequencies and at least one pair of electrical resistance values is measured at a measurement frequency that is different from a measurement frequency at which another pair of electrical resistance values is measured, and/or
- the muscle section (S) is determined from pairs of electrical resistance values measured at several different measurement frequencies and at least one pair of electrical resistance values is measured at a measurement frequency that is different from each measurement frequency at which each other respective pair of electrical resistance values is measured, and/or
- the muscle section is determined either from one pair of electrical resistance values or from several pair of electrical resistance values as appropriate, and/or
- the muscle section is determined either from electrical resistance values measured at a single measurement frequency or from electrical resistance values measured at several different measurement frequencies as appropriate, and/or
- the first or the second locations of the thigh at which respectively the first and the second electrical resistance values of a pair of electrical resistance values are measured is identical to a first or a second locations of the thigh at which respectively a first and a second electrical resistance values of another pair of electrical resistance values are measured, and/or
- the muscle section is determined solely from or as a function of, for each considered electrical resistance value, a product of the considered electrical resistance value and the permittivity constant of the muscle, and/or

- the muscle section is determined solely from or as a function of, for each considered electrical resistance value, a ratio between the considered electrical resistance value and the muscle conductivity constant, and/or
- the muscle section is determined solely from or as a function of a term of conductivity comprising, for each considered electrical resistance value, a product of the considered electrical resistance value and the muscle conductivity constant, and/or
- the corrective term is the product of the square of the measurement frequency and the square permittivity constant of the muscle, and/or
- the corrective term is, for each considered electrical resistance value, a product of the considered electrical resistance value and the square permittivity constant of the muscle, and/or
- the corrective term is, for each considered electrical resistance value, a ratio of the considered electrical resistance value and the muscle conductivity constant, and/or
- the corrective term is, for each considered electrical resistance value, a product of the considered electrical resistance value and the square of the measurement frequency, and/or
- the muscle section is determined as a function of the sum of the term of conductivity and the corrective term, and/or
- the invention relates to an apparatus comprising technical means arranged for and/or configured to and/or programmed to carry out the method according to invention, and/or
- the invention relates to a computer program product comprising instructions which, when the program is executed by a computer, cause the computer to carry out the method according to the invention.

**Claims**

1.  Computer-implemented method for determining a muscle section of a body part in which the muscle section (S) is determined as a function of:

    - at least two electrical resistance values measured through different electrodes located on different locations of the body, at least two electrodes being located along said body part, and
    - a muscle conductivity constant ($\sigma$), and
    - a permittivity constant of the muscle ($\varepsilon$),
    the muscle section (S) is determined as a function of formula 1,

    $$S = \frac{1}{\frac{\delta R}{\delta z} \cdot (\sigma + \frac{\varepsilon^2 w^2}{\sigma})}, \text{ formula 1,}$$

    in which $\frac{\delta R}{\delta z}$ is an electrical resistance gradient between two locations of the body part separated from a distance z and $\omega$ is the pulsation at which the at least two electrical resistance values are measured, the electrical resistance gradient $\frac{\delta R}{\delta z}$ being determined from said at least two electrical resistance values.

2.  Method according to claim 1, in which the muscle section is determined from:

    - at least a pair of electrical resistance values being measured at a single measurement frequency or at different measurement frequencies;

    each pair of electrical resistance values comprising a first electrical resistance value measured at a first electrode located at a first location of the body part and a second electrical resistance value measured at a second electrode located at a second location of the body part.

3.  Method according to the preceding claim, in which the first or the second locations of the body part at which respectively the first and the second electrical resistance values of a pair of electrical resistance values are measured is identical to a first or a second locations of the body part at which respectively a first and a second electrical resistance values of another pair of electrical resistance values are measured.

4.  Method according to any of the preceding claims, in which the muscle conductivity constant and/or the permittivity constant of the muscle are a function of the measurement frequency at which the at least two electrical resistance values are measured; said muscle conductivity constant being comprises between 0.1 and 2 S/m, preferably between 0.4 and 1.5 S/m, more preferably between 0.6 and 1.2 S/m; said permittivity constant of the muscle being comprises between $1.10^{-8}$ and $1.10^{-6}$ F/m, more preferably between $5.10^{-8}$ and $7.10^{-7}$ F/m.

5.  Method according to any of the preceding claims, in which:

    - the muscle conductivity constant is defined as a function of the measurement frequency f according to formula 2:

$$\sigma = af^2 + bf + c, \text{ formula 2,}$$

    and/or
    - the permittivity constant is defined as a function of the measurement frequency f according to formula 3:

$$\varepsilon = d.e^{-gf} + h.e^{-kf}, \text{ formula 3}$$

    where the terms a, b, c, d, g, h and k of the equations are constants.

6.  Method according to any of the preceding claims, in which the measurement frequency is comprised between 40 and 1000 kHz.

7.  Method for determining a muscle volume of a body part, in which the muscle volume of the body part is determined from one or more muscle sections of the body part, each muscle section of the body part being determined according to any of claims 1 to 6.

8.  Apparatus comprising technical means arranged for and/or configured to and/or programmed to carry out the method according to any of claims 1 to 6.

9.  Computer program product comprising instructions which, when the program is executed by a computer, cause the computer to carry out the method according to any of claims 1 to 6.


**Patentansprüche**

1.  Computerimplementiertes Verfahren zum Bestimmen eines Muskelabschnitts eines Körperteils, wobei der Muskelabschnitt (S) als eine Funktion bestimmt wird von:

    - mindestens zwei elektrischen Widerstandswerten, die durch verschiedene Elektroden gemessen werden, die an verschiedenen Stellen des Körpers gelegen sind, wobei mindestens zwei Elektroden entlang des Körperteils gelegen sind, und
    - einer Muskelleitfähigkeitskonstante ($\sigma$), und
    - einer Permittivitätskonstante des Muskels ($\varepsilon$),
    wobei der Muskelabschnitt (S) als eine Funktion von Formel 1 bestimmt wird,

$$S = \frac{1}{\frac{\delta R}{\delta z}.(\sigma + \frac{\varepsilon^2 w^2}{\sigma})}, \text{ Formel 1,}$$

    wobei $\frac{\delta R}{\delta z}$ ein elektrischer Widerstandsgradient zwischen zwei Stellen des Körperteils ist, die um eine Entfernung z voneinander getrennt sind, und $\omega$ die Pulsation ist, bei der die mindestens zwei elektrischen Widerstandswerte gemessen werden, wobei der elektrische Widerstandsgradient $\frac{\delta R}{\delta z}$ aus den mindestens zwei elektrischen Widerstandswerten bestimmt wird.

2.  Verfahren nach Anspruch 1, wobei der Muskelabschnitt bestimmt wird aus:

- mindestens einem Paar von elektrischen Widerstandswerten, die bei einer einzigen Messfrequenz oder bei verschiedenen Messfrequenzen gemessen werden; jedes Paar von elektrischen Widerstandswerten umfassend einen ersten elektrischen Widerstandswert, der an einer ersten Elektrode gemessen wird, die an einer ersten Stelle des Körperteils gelegen ist, und einen zweiten elektrischen Widerstandswert, der an einer zweiten Elektrode gemessen wird, die an einer zweiten Stelle des Körperteils gelegen ist.

3. Verfahren nach dem vorstehenden Anspruch, wobei die erste oder die zweite Stelle des Körperteils, an der jeweils der erste und der zweite elektrische Widerstandswert eines Paars von elektrischen Widerstandswerten gemessen werden, identisch ist mit einer ersten oder einer zweiten Stelle des Körperteils, an der jeweils der erste und der zweite elektrische Widerstandswert eines anderen Paars von elektrischen Widerstandswerten gemessen werden.

4. Verfahren nach einem der vorstehenden Ansprüche, wobei die Muskelleitfähigkeitskonstante und/oder die Permittivitätskonstante des Muskels eine Funktion der Messfrequenz sind, bei der die mindestens zwei elektrischen Widerstandswerte gemessen werden; wobei die Muskelleitfähigkeitskonstante zwischen 0,1 und 2 S/m, vorzugsweise zwischen 0,4 und 1,5 S/m, mehr bevorzugt zwischen 0,6 und 1,2 S/m umfasst ist; wobei die Permittivitätskonstante des Muskels zwischen $1{,}10^{-8}$ und $1{,}10^{-6}$ F/m, mehr bevorzugt zwischen $5{,}10^{-8}$ und $7{,}10^{-7}$ F/m umfasst ist.

5. Verfahren nach einem der vorstehenden Ansprüche, wobei:

- die Muskelleitfähigkeitskonstante als eine Funktion der Messfrequenz f gemäß Formel 2 definiert ist:

$$\sigma = af^2 + bf + c, \text{ Formel 2,}$$

und/oder
- die Permittivitätskonstante als eine Funktion der Messfrequenz f gemäß Formel 3 definiert ist:

$$\varepsilon = d.\ e^{-gf} + h.\ e^{-kf}, \text{ Formel 3}$$

wobei die Terme a, b, c, d, g, h und k der Gleichungen Konstanten sind.

6. Verfahren nach einem der vorstehenden Ansprüche, wobei die Messfrequenz zwischen 40 und 1000 kHz umfasst ist.

7. Verfahren zum Bestimmen eines Muskelvolumens eines Körperteils, wobei das Muskelvolumen des Körperteils aus einem oder mehreren Muskelabschnitten des Körperteils bestimmt wird, wobei jeder Muskelabschnitt des Körperteils nach einem der Ansprüche 1 bis 6 bestimmt wird.

8. Vorrichtung, umfassend technische Mittel, die angeordnet sind für und/oder konfiguriert sind um und/oder programmiert sind, um das Verfahren nach einem der Ansprüche 1 bis 6 auszuführen.

9. Computerprogrammprodukt, umfassend Anweisungen, die, wenn das Programm durch einen Computer durchgeführt wird, den Computer veranlassen, das Verfahren nach einem der Ansprüche 1 bis 6 auszuführen.

**Revendications**

1. Procédé mis en œuvre par ordinateur pour déterminer une section musculaire d'une partie du corps, ladite section musculaire (S) étant déterminée en fonction :

- d'au moins deux valeurs de résistance électrique mesurées au moyen de différentes électrodes disposées en des emplacements distincts du corps, au moins deux électrodes étant disposées le long de ladite partie du corps, and
- d'une constante de conductivité musculaire ($\sigma$), and
- d'une constante de permittivité du muscle ($\varepsilon$),
la section musculaire (S) étant déterminée en fonction de la formule 1,

$$S = \frac{1}{\frac{\delta R}{\delta z} \cdot (\sigma + \frac{\varepsilon^2 w^2}{\sigma})}, \text{ formule 1,}$$

dans laquelle $\frac{\delta R}{\delta z}$ i est un gradient de résistance électrique entre deux emplacements de la partie du corps séparés par une distance z and $\omega$ est la pulsation à laquelle lesdites au moins deux valeurs de résistance électrique sont mesurées, le gradient de résistance électrique $\frac{\delta R}{\delta z}$ étant déterminé à partir desdites au moins deux valeurs de résistance électrique.

2. Procédé selon la revendication 1, dans lequel la section musculaire est déterminée à partir de :

- au moins une paire de valeurs de résistance électrique mesurées à une seule fréquence de mesure ou à des fréquences de mesure différentes ; chaque paire de valeurs de résistance électrique comprenant une première valeur de résistance électrique mesurée à une première électrode située à un premier emplacement de la partie du corps et une seconde valeur de résistance électrique mesurée à une seconde électrode située à un second emplacement de la partie du corps.

3. Procédé selon la revendication précédente, dans lequel les premier ou second emplacements de la partie du corps auxquels sont respectivement mesurées la première et la seconde valeurs de résistance électrique d'une paire de valeurs de résistance électrique sont identiques aux premier ou second emplacements de la partie du corps auxquels sont respectivement mesurées une première et une seconde valeurs de résistance électrique d'une autre paire de valeurs de résistance électrique.

4. Procédé selon l'une quelconque des revendications précédentes, dans lequel la constante de conductivité musculaire et/ou la constante de permittivité du muscle sont fonction de la fréquence de mesure à laquelle lesdites au moins deux valeurs de résistance électrique sont mesurées ; ladite constante de conductivité musculaire étant comprise entre 0,1 et 2 S/m, de préférence entre 0,4 et 1,5 S/m, plus préférentiellement entre 0,6 et 1,2 S/m ; ladite constante de permittivité du muscle étant comprise entre $1 \times 10^{-8}$ et $1 \times 10^{-6}$ F/m, plus préférentiellement entre $5 \times 10^{-8}$ et $7 \times 10^{-7}$ F/m.

5. Procédé selon l'une quelconque des revendications précédentes, dans lequel :

- a constante de conductivité musculaire est définie en fonction de la fréquence de mesure f selon la formule 2 :

$$\sigma = af^2 + bf + c, \text{ formule 2,}$$

et/ou
- la constante de permittivité est définie en fonction de la fréquence de mesure f selon la formule 3 :

$$\varepsilon = d.e^{-gf} + h.e^{-kf}, \text{ formule 3}$$

où les termes a, b, c, d, g, h et k des équations sont des constantes.

6. Procédé selon l'une quelconque des revendications précédentes, dans lequel la fréquence de mesure est comprise entre 40 et 1000 kHz.

7. Procédé de détermination d'un volume musculaire d'une partie du corps, dans lequel le volume musculaire de la partie du corps est déterminé à partir d'une ou de plusieurs sections musculaires de ladite partie du corps, chaque section musculaire de la partie du corps étant déterminée selon l'une quelconque des revendications 1 à 6.

8. Dispositif comprenant des moyens techniques agencés pour et/ou configurés pour et/ou programmés pour mettre en œuvre le procédé selon l'une quelconque des revendications 1 à 6.

9. Produit programme d'ordinateur comprenant des instructions qui, lorsque le programme est exécuté par un ordinateur, amènent l'ordinateur à mettre en œuvre le procédé selon l'une quelconque des revendications 1 à 6.

Fig. 1

Fig. 2

Fig. 3

Fig. 4

Fig. 5

Fig. 6

Fig. 7

Fig. 8

**EP 3 788 957 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SALINARI et al.** *Journal of Applied Physiology*, 2003, vol. 94, 1552-1556

- **SALINARI et al.** *American Journal of Physiology Endocrinology and Metabolism*, 2002, vol. 282, E960-6